# EUROPEAN PATENT SPECIFICATION

(11) **EP 1 535 623 B1**
(45) Date of publication and mention of the grant of the patent: **31.12.2008**
(21) Application number: 05002952.9
(22) Date of filing: 06.04.2000
(51) Int. Cl.: A61K 38/21, A61K 47/48, A61P 35/00

(54) **Renal cell carcinoma treatment**
Behandlung von Nierenzellen-Karzinom
Traitement du carcinome de cellules rénales

(30) Priority: 08.04.1999 US 288359
(43) Date of publication of application: 01.06.2005
(62) Divisional of application: 00107100.0
(73) Proprietor: Schering Corporation, Kenilworth, New Jersey 07033 (US)
(72) Inventor: Rose, Esther Helen, Westfield NJ 07090 (US); Rybak, Mary Ellen, Warren NJ 07059 (US)
(74) Representative: UEXKÜLL & STOLBERG

(56) References cited:
- WO-A-98/48840
- WO-A-99/48535
- US-A- 5 766 582
- ATZPODIEN JENS ET AL: "Interleukin-2 in combination with interferon-alpha and 5-fluorouracil for metastatic renal cell cancer." EUROPEAN JOURNAL OF CANCER, vol. 29A, no. SUPPL. 5, 1993, pages S6-S8, XP000953180 ISSN: 0959-8049
- ATZPODIEN JENS ET AL: "Multiinstitutional Home-Therapy Trial of Recombinant Human Interleukin-2 and Interferon Alfa-2 in Progressive Metastatic Renal Cell Carcinoma." JOURNAL OF CLINICAL ONCOLOGY, vol. 13, no. 2, 1995, pages 497-501, XP000953198 ISSN: 0732-183X
- GEBROSKY NORMAN P ET AL: "Treatment of renal cell carcinoma with 5-fluorouracil and alfa-interferon." UROLOGY, vol. 50, no. 6, December 1997 (1997-12), pages 863-868, XP000953183 ISSN: 0090-4295
- WALTHER PHILIP J ET AL: "Treatment of metastatic renal cell carcinoma (RCC) with continuous infusion 5-fluorouracil and interferon alpha-2A in the home setting: A phase I-II trial." JOURNAL OF UROLOGY, vol. 155, no. 5 SUPPL., 1996, page 388A, XP000953188 ISSN: 0022-5347
- TALPAZ M ET AL: "Phase I study of polyethylene glycol (PEG) interferon alpha-2B (Intron-A) in CML patients." BLOOD, vol. 92, no. 10 SUPPL. 1 PART 1-2, 15 November 1998 (1998-11-15), page 251A, XP000953118 ISSN: 0006-4971
- TALPAZ M ET AL: "Phase I study of pegylated-interferon alpha-2a (PEGASYSTM) in patients with chronic myelogenous leukemia (CML)." BLOOD, vol. 94, no. 10 SUPPL. 1 PART 1, 15 November 1999 (1999-11-15), page 530a, XP000953172 ISSN: 0006-4971
- BUKOWSKI RONAL ET AL: "Phase I Study of Polyethylene Glycol ( PEG ) Interferon Alpha -2B ( PEG INTRON ) in Patients with Solid Tumors (Meeting abstract)." PROC ANNU MEET AM SOC CLIN ONCOL, vol. 18, May 1999 (1999-05), page A1719, XP000953163

## Description

### BACKGROUND OF THE INVENTION

This invention relates to an improved therapy for treating patients having renal cell carcinoma ("RCC") by administering a therapeutically effective dose of pegylated interferon-alfa for a time sufficient to achieve at least a partial tumor response.

Metastatic renal cell carcinomas are generally resistant to chemotherapy, either with single agents or in combination. Greater success has been seen with immunotherapy, particularly with the use of interleukin-2 ("IL-2"). Therapy with high dose intravenous IL-2 has resulted in objective tumor responses in approximately 14% of patients, some with long durability. The administration of high dose IL-2 is associated with capillary leak syndrome, which results in hypotension and reduced organ perfusion which can be severe and sometimes fatal. These toxicities have generally inhibited the use of IL-2 to a highly selected group of patients administered by physicians with significant experienced in its administration. The use of lower-dose and subcutaneously (SC) administered regimens of IL-2 alone or in combination with other biologic agents, including interferon-α, has been explored in an effort to develop a more broadly applicable therapy for this disease. For example, Atzpodien, J., et al. disclosed in J. Clin Oncol., 1995, Volume 13, pages 497-501 that the combination treatment of subcutaneous ("SC") administration and lower dose regimens of interferon alfa-2b and SC interleukin-2 to patients with progressive metastatic RCC produced tumor response with lower toxicity. It must be noted that the response rates are low and that injections of interferon alfa-2b 5 miiiom IU/m^{2,} three times a week ("TIW"), were required to achieve these results.

In addition, interferon alfa-2b has many side effects that a substantial number of patients find unacceptable, and patient compliance with the TIW injections of interferon alfa-2b has become a problem. WO 98/48840 describes polyethylene glycol-interferon alpha conjugates that are useful in methods of treating viral infections, particularly hepatitis C, but does not disclose using these conjugates to treat RCC. Accordingly, there is a need for an improved therapy for treating patients having RCC.

### Summary of the Invention

The present invention provides a method of treating a patient having renal cell carcinoma which comprises administering to such a patient a therapeutically effective dose of pegylated interferon alfa for a time period sufficient to effect at least a partial tumor response.

The present invention also provides a method of treating a patient having metastatic renal cell carcinoma which comprises administering to said patient an effective amount of pegylated interferon-alfa once a week for a time period sufficient to effect at least a partial tumor response.

The present invention further provides a method of treating a patient having metastatic renal cell carcinoma which comprises administering to such a patient about 4.5 micrograms/kg to about 9.0 micrograms/kg of pegylated interferon alfa-2b once a week for a time period sufficient to effect at least a partial tumor response.

### Detailed Description of the Invention

The present invention provides an improved method of treating patients with RCC-especially those with metastatic RCC. The improved method provides a safer and more efficacious and tolerable treatment for RCC by use of weekly injections of pegylated interferon alfa alone or in combination with immunotherapeutic agents such as IL-2 or fluorouracil (5-FU").The RCC patients include those newly diagnosed with this disease as well as those patients intolerant or resistant to interferon alfa. Treatment with pegylated interferon alfa in accordance with the present invention will continue for a minimum of six months, and preferably for at least twelve months unless there is clinical evidence of disease progression, unacceptable toxicity or the patient requests that the therapy be discontinued.

When the pegylated interferon-alfa administered is a pegylated interferon alfa-2b, the therapeutically effective amount of pegylated interferon alfa-2b administered is in the range of about 4.5 to about 9.0 micrograms per kilogram of pegylated interferon alfa-2b administered once a week (QW), preferably in the range of about 4.5 to about 6.5 micrograms per kilogram of pegylated interferon alfa-2b QW, more preferably in the range of about 5.5 to about 6.5 micrograms per kilogram of pegylated interferon alfa-2b QW, and most preferably in the range of about 6.0 micrograms per kilogram of pegylated interferon alfa-2b administered QW.

When the pegylated interferon-alfa administered is a pegylated interferon alfa-2a, the therapeutically effective amount of pegylated interferon alfa-2a administered is in the range of about 50 micrograms to about 500 micrograms once a week("QW"), preferably about 200 micrograms to about 250 micrograms QW.

The term "pegylated interferon alfa" as used herein means polyethylene glycol modified conjugates of interferon alfa, preferably interferon alfa-2a and -2b. The preferred polyethylene-glycol-interferon alfa -2b conjugate is PEG₁₂₀₀₀-interferon alfa 2b. The phrases "12,000 molecular weight polyethylene glycol conjugated interferon alpha" and "PEG₁₂₀₀₀-IFN alfa" as used herein mean conjugates such as are prepared according to the methods of International Application No. WO 95/13090 and containing urethane linkages between the interferon alfa-2a or -2b amino groups and polyethylene glycol having an average molecular weight of 12000.

The preferred PEG₁₂₀₀₀-interferon alfa-2b is prepared by attaching a PEG polymer to the epsilon amino group of a lysine residue in the IFN alfa-2b molecule. A single PEG₁₂₀₀₀ molecule is conjugated to free amino groups on an IFN alfa-2b molecule via a urethane linkage. This conjugate is characterized by the molecular weight of PEG₁₂₀₀₀ attached. The PEG₁₂₀₀₀-IFN alfa-2b conjugate is formulated as a lyophilized powder for injection. The objective of conjugation of IFN alfa with PEG is to improve the delivery of the protein by significantly prolonging its plasma half-life, and thereby provide protracted activity of IFN alfa.

The term " interferon-alfa " as used herein means the family of highly homologous species-specific proteins that inhibit viral replication and cellular proliferation and modulate immune response. Typical suitable interferon-alfas include, but are not limited to, recombinant interferon alfa-2b such as Intron-A interferon available from Schering Corporation, Kenilworth, N.J., recombinant interferon alfa-2a such as Roferon interferon available from Hoffmann-La Roche, Nutley, N.J., recombinant interferon alpha-2C such as Berofor alpha 2 interferon available from Boehringer Ingelheim Pharmaceutical, Inc., Ridgefield, CT., interferon alpha-n1, a purified blend of natural alfa interferons such as Sumiferon available from Sumitomo, Japan or as Wellferon interferon alpha-n1 (INS) available from the Glaxo-Wellcome Ltd., London, Great Britain, or a consensus alpha interferon such as those described in U.S. Patent Nos. 4,897,471 and 4,695,623 (especially Examples 7, 8 or 9 thereof) and the specific product available from Amgen, Inc., Newbury Park, CA, or interferon alfa-n3 a mixture of natural alfa interferons made by Interferon Sciences and available from the Purdue Frederick Co., Norwalk, CT., under the Alferon Tradename. The use of interferon alfa-2a or alpha-2b is preferred. Since interferon alpha-2b, among all interferons, has the broadest approval throughout the world for treating chronic hepatitis C infection, it is most preferred. The manufacture of interferon alpha-2b is described in U.S. Patent No. 4,530,901.

Other interferon alfa conjugates can be prepared by coupling an interferon alfa to a water-soluble polymer. A non-limiting list of such polymers include other polyalkylene oxide homopolymers such as polypropylene glycols, polyoxyethylenated polyols, copolymers thereof and block copolymers thereof. As an alternative to polyalkylene oxide-based polymers, effectively non-antigenic materials such as dextran, polyvinylpyrrolidones, polyacrylamides, polyvinyl alcohols, carbohydrate-based polymers and the like can be used. Such interferon alfa-polymer conjugates are described in U.S. Patent No. 4,766,106, U.S. Patent No. 4,917,888, European Patent Application No. 0 236 987, European Patent Application Nos. 0 510 356 , 0 593 868 and 0 809 996 (pegylated interferon alfa-2a) and International Publication No. WO 95/13090.

Pharmaceutical composition of pegylated interferon alfa-suitable for parenteral administration may be formulated with a suitable buffer, e.g., Tris-HCl, acetate or phosphate such as dibasic sodium phosphate/monobasic sodium phosphate buffer, and pharmaceutically acceptable excipients ( e.g., sucrose), carriers (e.g. human serum albumin), toxicity agents (e.g. NaCl), preservatives (e.g. thimerosol, cresol or benylalcohol), and surfactants( e.g. tween or polysorabates) in sterile water for injection. The pegylated interferon alfa-may be stored as lyophilized powders under a refrigeration at 2°-8°C. The reconstituted aqueous solutions are stable when stored between 2° and 8°C and used within 24 hours of reconstitution. See for example U.S. Patent Nos, 4,492,537; 5,762,923 and 5,766,582.The reconstituted aqueous solutionsmay also be stored in prefilled, multi-dose syringes such as those useful for delivery of drugs such as insulin. Typical suitable syringes include systems comprising a prefilled vial attached to a pen-type syringe such as the NOVOLET Novo Pen available from Novo Nordisk, as well as prefilled, pen-type syringes which allow easy self-injection by the user. Other syringe systems include a pen-type syringe comprising a glass cartridge containing a diluent and lyophilized pegylated interferon alfa powder in a separate compartment.

The term "tumor response" as used herein means a reduction or elimination of all measurable lesions.

The criteria for tumor response are based on the WHO Reporting Criteria [WHO Offset Publication, 48-World Health Organization, Geneva, Switzerland, (1979)]. Ideally, all uni-or bidimensionally measurable lesions should be measured at each assessment. When multiple lesions are present in any organ, such measurements may not be possible and, under such circumstances, up to 6 representative lesions should be selected, if available.

The term "complete response ("CR") as used herein means a complete disappearance of all clinically detectable malignant disease, determined by 2 observations not less than 4 weeks apart. A preliminary assessment may be made with two measurements.

The term "partial response" ("PR") as used herein in reference to (a) bidimensionally measurable disease means decrease by at least about 50% of the sum of the products of the largest perpendicular diameters of all measurable lesions as determined by 2 observations not less than 4 weeks apart and (b) unidimensionally measurable disease means decrease by at least about 50% in the sum of the largest diameters of all lesions as determined by 2 observations not less than 4 weeks apart. It is not necessary for all lesions to have regressed to qualify for partial response, but no lesion should have progressed and no new lesion should appear. Serial evidence of appreciable change documented by copies of radiographic studies must be obtained and must be available for subsequent review. The assessment should be objective.

The term "stable disease" ("SD") as used herein in reference to (a) bidimensionally measurable disease means less than about 50% decrease or less than about 25% increase in the sum of the products of the largest perpendicular diameters of all measurable lesions and (b) unidimensionally measurable disease, means less than about 50% decrease or less than about 25% increase in the sum of the diameters of all lesions. No new lesions should appear.

The term "progressive disease ("PD") as used herein means a greater than or equal to about a 25% increase in the size of at least one bidimensionally (product of the largest perpendicular diameters) or unidimensionally measurable lesion or appearance of a new lesion. The occurrence of pleural effusion or ascites is also considered as progressive disease if this is substantiated by positive cytology. Pathological fracture or collapse of bone are not necessarily evidence of disease progression.

### Overall Subject Tumor Response

Determination of overall subject tumor response for uni- and bidimensionally meaurable disease will be done according to the following table:

**Overall Subject Tumor Response**

| Response in Bidimensionally Measurable Disease | Response in Unidimensionally Measurable Disease | Overall Subject Tumor Response |
|---|---|---|
| PD | Any | PD |
| Any | PD | PD |
| SD | SD or PR | SD |
| SD | CR | PR |
| PR | SD or PR or CR | PR |
| CR | SD or PR | PR |
| CR | CR | CR |

| | | |
|---|---|---|
| PD: Progressive Disease. CR: Complete Response. PR: Partial Response. SD: Stable Disease | | |

### Evaluation of Response in the Presence of Non-Measurable Disease

Non-measurable disease will not be used in the assessment of overall subject tumor response except in the following situations:
a) **Overall complete response:** if non-measurable disease is present, it should disappear completely. Otherwise, the subject cannot be considered as an "overall complete responder".
b) **Overall progression:** in case of a significant increase in the size of non-measurable disease or the appearance of a new lesion,
the overall response will be progression.

The term "patients having renal cell carcinoma or "RCC" as used herein means any patient having RCC and includes treatment-naive patients as well as treatment-experienced patients as well as patients in the metastatic stage of RCC.

The term "treatment-naive patients" as used herein means patients with RCC-including newly-diagnosed RCC patients- who have never been treated with radiation therapy or any chemotherapy, e.g., fluorouracil ("5-FU") or hormonal therapy or immunotherapy, e.g. IL-2, as well as any interferon, including but not limited to interferon alfa, or pegylated interferon alfa.

The term "treatment-experienced patients" as used herein means those patients who have initiated some form of radiation therapy or hormonal therapy or chemotherapy including, but not limited to 5 FU, or immunotherapy including, but not limited to IL-2. Interleukin-2 is available under the PROLEUKIN® tradename from Chiron Corporation, Emeryville, CA 94608-3997. Fluorouracil or 5-FU is availableas an injectable solution under the ADRUCIL® tradename from Pharmacia & UpjohnCo., Bridgewater, NJ 08807-12665

The effective amount of IL-2 when used is in the range of about 5 to about 20 million international units ("IU") per square meter of body surface area (m²) three times per week ("TIW") administered subcutaneously. In a preferred embodiment of the method of the present invention, the dosage and dosage regimen for SC administration of IL-2 in combination with pegylated interferon alfa will be about 20 million IU/m² TIW in the first week and every fourth week thereafter and about 5 million IU/m² TIW in the subsequent weeks of treatment, e.g., weeks 2, 3, 5, 6, 7, 9, 10, 11 et seq.

The effective amount of 5-FU when used is in the range of about 12 mg/kg/day IV( not to exceed 800mg) for 5 days. If toxicity has not occurred, 6 mg/kg/day IV on days 7, 9, 11 and 13.For maintenance,mif toxicity to the first course was minal either repeat the course every 30 days or give 10 to 15 mg/kg (not to exceed 1 g ) once a week, after recovery from the initial toxicity is complete.These doses may be reduced by the clinician depending upon the severity of the disease and the patient's condition and reaction to pegylated interferon alfa. A constant infusion with an implantable pump may be more effective and fewer toxic than periodic bolus injections.

Pegylated interferon-alfa formulations are not effective when administered orally, so the preferred method of administering the pegylated interferon-alfa is parenterally, preferably by subcutaneous, IV, or IM, injection. Of course, other types of administration of both medicaments, as they become available are contemplated, such as by nasal spray, transdermally, by suppository, by sustained release dosage form, and by pulmonary inhalation. Any form of administration will work so long as the proper dosages are delivered without destroying the active ingredient.

The following Clinical Study Design may be used to treat RCC patients in accordance with the method of the present invention. Many modifications of this Clinical Study Design protocol will be obvious to the skilled clinician, and the following Study Design should not be interpreted as limiting the scope of the method of this invention which is defined by the claims listed hereinafter

### Clinical Study Design

In a preferred embodiment of the treatment method of the present invention, subjects with metastatic RCC will receive pegylated interferon alfa 2b, i.e., PEG₁₂₀₀₀-interferon alfa 2b at doses of 6.0 micrograms per kilogram by subcutaneous injection once a week.

### Duration of Study and Visit Schedule

The duration of this study is based upon achieveing a therapeutic response, and will be determined for each subject individually.

Treatment with PEG Intron will continue for a minimum of 6 months unless there is evidence of disease progression, unacceptable toxicity, or the subject requests that therapy be discontinued. Tumor response will be assessed beginning at week 8 will be evaluated every 8 weeks thereafter during the first year of study treatment. Population pharmacokinetics will be conducted at various timepoints throughout the study. In addition, quality of life and overall survival data will be collected. Subjects who achieve a complete or partial tumor response by 6 months will continue treatment for another 6 months.

The following clinical protocol may be used to administer the RCC therapy of the present invention:

The study population will include male and female patients with metastatic RCC and will be included if they meet the following inclusion and exclusion criteria:

### Subject Inclusion Criteria

A subject is eligible to participate in this study if he or she:
a) has histologically documented metastic renal cell carcinoma.
b) has an ECOG Performance Status of 0 or 1.
c) is ≥18 and ≤70 years of age.
d) has a life expectancy of >8 weeks.
e) has adequate end organ function (hepatic, renal, bone marrow, cardiac) as indicated by laboratory values below:
   1) Hematology:
      - Absolute neutrophil count (ANC) ≥3,000 cells/µL.
      - Platelet count ≥100,000 cells/µL.
      - Hemoglobin concentration ≥9g/dL.
   2) Renal and hepatic function:
      - Serum creatinine ≤1.8 mg/dL or calculated creatinine clearance of ≥50 mL/minute.
      - Serum bilirubin ≤1.25 times the upper limit of normal (ULN), unless due to infiltration by disease.
      - AST/ALT (SGOT/SGPT) ≤1.25 times ULN, unless due to infiltration by disease.
      - Negative HBs Ag.
   3) Normal plasma calcium.
f) has submitted a written voluntary informed consent before study entry, is willing to participate in this study and will complete all follow up assessments.

### Subject Exclusion Criteria

A subject is not eligible to participate in this study if he or she:
a) has received any prior non-surgical treatment for RCC, including chemotherapy, adjuvant chemotherapy, immunotherapy, radiation therapy or hormonal therapy.
b) has evidence of CNS metastases.
c) has a known hypersensitivity to interferon-α.
d) has severe cardiovascular disease i.e., arrhythmias requiring chronic treatment or congestive heart failure (NYHA classification III or IV)
e) has a history of a neurophychiatric disorder requiring hospitalization.
f) has a history of seizure disorder.
g) has thyroid dysfunction not responsive to therapy.
h) has uncontrolled diabetes mellitus.
i) has a life-threatening second active malignancy.
j) has an ongoing active infection requiring antibiotics.
k) requires chronic treatment with systemic corticosteroids.
l) has a history of seropositivity for HIV.
m) is pregnant, lactating or of child-bearing potential and not practicing an effective means of contraception.
n) has active hepatitis.
o) is known to be actively abusing alcohol or drugs.
p) has received any experimental therapy within 30 days of enrollment into this study. and
q) has not recovered from the effects of any recent surgery.

### Subject Discontinuation Criteria

It is the right and duty of the clinical investigator to interrupt the treatment of any subject whose health or well being may be threatened by continuation in this study.

A subject may be discontinued prior to completion of the study if he or she:
a) has a clinically significant adverse event as determined by the Principal Investigator.
b) requests to be withdrawn from the study.
c) is unable to complete the study evaluations/visits because of unforeseen circumstances.
d) develops other conditions for which, in the investigator's opinion, it is in the subject's best interest to be withdrawn from the study.
e) develops severe depression or any other psychiatric disorder requiring hospitalization.
f) experiences a serious allergic response to the study drug manifested by angioedema, bronchoconstriction or anaphylaxis.

### Another Clinical Study Design

In another preferred embodiment of the treatment method of the present invention, subjects with metastatic RCC will receive pegylated interferon alfa 2b, i.e., PEG₁₂₀₀₀-interferon alfa 2b at doses of 6.0 micrograms per kilogram by subcutaneous injection once a week in combination with SC administration of IL-2 in accordance with the following regimen: 20 million IU of IL-2/M² TIW in week 1 and every fourth week thereafter and 5 million IU of IL-2/M² TIW in weeks 2, 3, 5, 6, 7, 9, 10, 11 et seq.

### Analysis of Primary and Secondary Endpoints

The primary efficacy endpoint will be the tumor response at 6 months. The primary analysis will be the comparison of treatment groups with respect to the proportion of subjects with major tumor response at 6 months using the Cochran Mantel-Haenszel test adjusting for strata. Odds ratio and 95% confidence intervals for the odds ratio will be summarized.

The secondary endpoints of the study will be relapse-free survival at 3, 6 and 12 months, and overall survival. Relapse-free survival and overall survival will be analyzed using the log-rank statistic. Kaplan-Meier estimates of the survival curves will be provided. Hazar ratio and 95% confidence interval for the hazard ratio will be obtained using Cox's proportional hazards model.

## Claims

1. The use of pegylated interferon alpha-2b for the manufacture of a medicament for treatment of a patient having renal cell carcinoma, wherein said medicament is to be administered in a therapeutically effective dose of pegylated interferon alpha-2b once a week for a period of at least 12 month, wherein the pegylated interferon alpha-2b comprises polyethylene glycol having an average molecular weight of 12,000 and the therapeutically effective dose is in the range of about 4.5 µg/kg to about 9.0 µg/kg.

2. The use according to claim 1, wherein the therapeutically effective dose is in the range of about 5.5 µg/kg to about 6.5 µg/kg.

3. The use according to claim 1, wherein the therapeutically effective dose is about 6.0 µg/kg.

4. The use according to claims 1-3, wherein said medicament is to be administered in the therapeutically effective dose once a week for a time period sufficient to effect at least a partial tumor response.

5. The use according to claims 1-4, wherein said medicament is to be administered in combination with subcutaneous administration of interleukin-2 (IL-2) in an amount of about 5 to about 20 million international units (IU) per square meter of body surface area (m²) three times per week (TIW).

6. The use according to claim 5, wherein the dosage and dosage regimen for the administration of IL-2 will be about 20 million IU/m² TIW in the first week and every fourth week thereafter and about 5 million IU/m² TIW in the subsequent weeks of treatment.

7. The use according to claims 1-4, wherein said medicament is to be administered in combination with intravenous administration of fluorouracil (5-FU) in an amount of about 12 mg/kg/day for 5 days.

8. The use according to claim 7 further comprising intravenous administration of 6 mg/kg/day 5-Fu on days 7, 9, 11 and 13.

9. The use according to claims 1-8, wherein the pegylated interferon alpha-2b is formulated as a lyophilized powder for injection.

10. The use according to claims 1-9, wherein the patient is a treatment-naive patient.

11. The use according to claims 1-9, wherein the patient is a treatment-experienced patient who is intolerant to interferon alpha or resistant to interferon alpha.

12. The use according to claims 4-11, wherein the tumor response is a complete tumor response.

13. The use according to claims 1-12, wherein the renal cell carcinoma is metastatic renal cell carcinoma.

## Patentansprüche

1. Verwendung von pegyliertem Interferon alpha-2b zur Herstellung eines Arzneimittels zur Behandlung eines Patienten mit Nierenzellkarzinom, wobei das Medikament in einer therapeutisch wirksamen Dosis an pegyliertem Interferon alpha-2b einmal pro Woche für eine Dauer von mindestens zwölf Monaten zu verabreichen ist, wobei das pegylierte Interferon alpha-2b Polyethylenglykol umfaßt, welches ein durchschnittliches Molekulargewicht von 12.000 aufweist und die therapeutisch wirksame Dosis im Bereich von etwa 4,5 µg/kg bis etwa 9,0 µg/kg liegt.

2. Verwendung gemäß Anspruch 1, bei der die therapeutisch wirksame Dosis im Bereich von etwa 5,5 µg/kg bis etwa 6,5 µg/kg liegt.

3. Verwendung gemäß Anspruch 1, bei der die therapeutisch wirksame Dosis etwa 6,0 µg/kg ist.

4. Verwendung gemäß einem der Ansprüche 1-3, bei der das Medikament in der therapeutisch wirksamen Dosis einmal pro Woche für eine Dauer zu verabreichen ist, die ausreicht, um mindestens eine teilweise Tumorreaktion zu erreichen.

5. Verwendung gemäß einem der Ansprüche 1-4, bei der das Medikament in Kombination mit subkutaner Verabreichung von Interleukin-2 (IL-2) in einer Menge von etwa 5 bis etwa 20 Millionen Internationalen Einheiten (IU) pro Quadratmeter Körperoberfläche (m²) dreimal pro Woche (TIW) zu verabreichen ist.

6. Verwendung gemäß Anspruch 5, bei der die Dosis und das Behandlungsprotokoll zur Verabreichung des IL-2 etwa 20 Millionen IU/m² TIW in der ersten Woche und in jeder vierten Woche danach und etwa 5 Millionen IU/m² TIW in den nachfolgenden Wochen der Behandlung zu verabreichen ist.

7. Verwendung gemäß einem der Ansprüche 1-4, bei der das Medikament in Kombination mit intravenöser Verabreichung von Fluorouracil (5-FU) in einer Menge von etwa 12 mg/kg/Tag über fünf Tage zu verabreichen ist.

8. Verwendung gemäß Anspruch 7, welche ferner die intravenöse Verabreichung von 6 mg/kg/Tag 5-FU an Tag 7, 9, 11 und 13 umfaßt.

9. Verwendung gemäß einem der Ansprüche 1 bis 8, bei der das pegylierte Interferon alpha-2b als lyophilisiertes Pulver für die Injektion formuliert ist.

10. Verwendung gemäß den Ansprüchen 1 bis 9, bei der der Patient ein unbehandelter Patient ist.

11. Verwendung gemäß den Ansprüchen 1 bis 9, bei der der Patient ein zuvor behandelter Patient ist, der gegenüber Interferon alpha intolerant oder resistent ist.

12. Verwendung gemäß den Ansprüchen 4 bis 11, bei der die Tumorreaktion eine vollständige Tumorreaktion ist.

13. Verwendung gemäß den Ansprüchen 1-12, bei der das Nierenzellkarzinom ein metastasierendes Nierenzellkarzinom ist.

## Revendications

1. Utilisation d'un interféron pégylé alpha-2b pour la fabrication d'un médicament pour le traitement d'un patient ayant un carcinome cellulaire rénal, dans laquelle ledit médicament est à administrer à une dose thérapeutiquement efficace d'interféron alpha-2b pégylé une fois par semaine pendant une période d'au moins 12 mois, dans laquelle l'interféron alpha-2b pégylé comprend du polyéthylèneglycol ayant un poids moléculaire moyen de 12 000, et dans laquelle la dose efficace est de 4,5 µg/kg à environ 9 µg/kg.

2. Utilisation selon la revendication 1, dans laquelle la dose efficace thérapeutique est comprise entre environ 5,5 µg/kg à environ 6,5 µg/kg.

3. Utilisation selon la revendication 1, dans laquelle la dose efficace thérapeutique est d'environ 6 µg/kg.

4. Utilisation selon l'une quelconque des revendications 1 à 3, dans laquelle ledit médicament est administré à une dose efficace thérapeutique une fois par semaine pendant une période de temps suffisante pour obtenir au moins une réponse tumorale partielle.

5. Utilisation selon l'une quelconque des revendications 1 à 4, dans laquelle ledit médicament est à administrer en combinaison avec l'administration sous-cutanée d'interleukine-2 (IL-2) en une quantité d'environ 5 à environ 20 millions d'unités internationales (UI) par mètre carré de surface corporelle (m²) trois fois par semaine (TIW).

6. Utilisation selon la revendication 5, dans laquelle le dosage et le dosage thérapeutique pour l'administration de IL-2 sera d'environ 20 millions d'UI/m² de TIW au cours de la première semaine et des quatre semaines suivantes et d'environ 5 millions d'UI/m² de TIW au cours de semaines suivantes de traitement.

7. Utilisation selon l'une des revendications 1 à 4, dans laquelle ledit médicament est à administrer en combinaison avec l'administration intraveineuse de fluorouracil (5-FU) en une quantité d'environ 12 mg/kg/jour pendant 5 jours.

8. Utilisation selon la revendication 7 qui comprend en outre l'administration par voie intraveineuse de 6 mg/kg/jour de 5-fluorouracil aux jours 7, 9, 11 et 13.

9. Utilisation selon l'une quelconque des revendications 1 à 8, dans laquelle l'interféron pégylé alpha-2b est formulé en tant que poudre lyophilisée pour injection.

10. Utilisation selon l'une quelconque des revendications 1 à 9, dans laquelle le patient est un patient naïf pour le traitement.

11. Utilisation selon l'une des revendications 1 à 9, dans laquelle le patient est un patient ayant déjà été traité, qui est intolérant à l'interféron alpha ou résistant à l'interféron alpha.

12. Utilisation selon l'une quelconque des revendications 4 à 11, dans laquelle la réponse tumorale est une réponse tumorale complète.

13. Utilisation selon l'une quelconque des revendications 1 à 12, dans laquelle le carcinome cellulaire rénal est un carcinome cellulaire rénal métastatique.
